# EUROPÄISCHE PATENTANMELDUNG

(11) **EP 3 213 740 A1**
(43) Veröffentlichungstag der Anmeldung: **06.09.2017**
(21) Anmeldenummer: 17156640.9
(22) Anmeldetag: 17.02.2017
(51) Int. Cl.: A61K 8/34, A61K 8/44, A61Q 17/04

(54) **GLYCIN-HALTIGES SONNENSCHUTZMITTEL**

(30) Priorität: 02.03.2016 DE 102016203378
(71) Anmelder: Beiersdorf AG, 20253 Hamburg (DE)
(72) Erfinder: Fänger, Sabine, 22763 Hamburg (DE); Gronau-Horn, Annette, 22549 Hamburg (DE); Weinert, Katrin, 22763 Hamburg (DE)

(57) **Zusammenfassung**

Kosmetische Zubereitung enthaltend
a) ein oder mehrere UV-Lichtschutzfilter,
b) Glycin,
c) Ethylendiamintetraessigsäure (EDTA) und/oder dessen Natriumsalz,
d) ein oder mehrerer 1,2-Alkandiole.

## Beschreibung

Die vorliegende Erfindung betrifft eine kosmetische Zubereitung enthaltend ein oder mehrere UV-Lichtschutzfilter, Glycin, Ethylendiamintetraessigsäure (EDTA) und/oder dessen Natriumsalz sowie ein oder mehrerer 1,2-Alkandiole.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

So wird in jüngerer Zeit in diesen Zubereitungen verstärkt die Aminosäure Glycin eingesetzt, beispielsweise, um als Hautbefeuchtungsmittel einen pflegenden Effekt auf der Haut zu erzielen. Der Einsatz von Glycin hat jedoch den Nachteil, bei dünnflüssigen, versprühbaren Zubereitungen das Sprühbild dahingehend zu verändern, dass die durch den Sprühkopf erzeugten kleinen "Nebeltröpfchen" zu größeren Tropfen zusammenballen und die Zubereitungen nicht als fein verteilter Sprühnebel gleichmäßig auf der Haut auftreffen, sondern, gleichsam wie aus einer Wasserpistole geschossen, in Form eines mehr oder weniger zusammenhängenden Strahls. Aufgrund der Zusammenballung der Zubereitungsmasse kommt es nach dem Auftreffen auf der Haut dann schwerkraftbedingt zu einem heruntertropfen und zerfließen der Zubereitung. Diese negative Veränderung des Sprühbildes führte nach dem Stand der Technik dazu, dass der Einsatz von Glycin trotz seines beachtlichen kosmetischen Potentials, in sprühbaren kosmetischen Zubereitungen nach Möglichkeit vermieden wird.

Es war daher die Aufgabe der vorliegenden Erfindung, eine Glycin enthaltende, sprühbare kosmetische Zubereitung, insbesondere ein Sonnenschutzmittel, zu entwickeln, welches bei Einsatz herkömmlicher Sprühköpfe und -applikatoren zu einen gleichmäßig fein verteilten Sprühbild führt.

Überraschend gelöst wird die Aufgabe durch eine kosmetische Zubereitung enthaltend
a) ein oder mehrere UV-Lichtschutzfilter,
b) Glycin,
c) Ethylendiamintetraessigsäure (EDTA) und/oder dessen Natriumsalz,
d) ein oder mehrerer 1,2-Alkandiole.

Zwar ist dem Fachmann natürlich bekannt, dass das gesamte Sprühsystem einen Einfluss auf das Sprühbild hat, die Lösung der Aufgabe sollte jedoch durch die Zubereitung an sich erfolgen. Ein Sprühbildvergleich erfolgt damit jeweils unter Verwendung des gleichen Sprühapplikators.

Neben der Verbesserung des Sprühbildes haben die erfindungsgemäßen Zubereitungen darüber hinaus den überraschenden Vorteil, dass die Rezepturen zur Erzielung ihrer mikrobiellen Stabilität mit einer deutlich reduzierteren Menge an konservierend wirkenden Verbindungen auskommen als das nach dem Stand der Technik der Fall ist. Zwar sind dem Fachmann die konservierend wirkenden Eigenschaften der 1,2-Alkandiole durchaus bekannt. Die erfindungsgemäße Zusammensetzung wirkt in Ihrer Gesamtheit in dieser Hinsicht jedoch deutlich besser/stärker als dies beim Einsatz der 1,2-Alkandiole allein der Fall ist.

Die erfindungsgemäßen Zubereitungen sind bevorzugt dadurch gekennzeichnet, dass die Zubereitung sprühbar ist.

Als "sprühbar" wird eine Zubereitung erfindungsgemäß insbesondere dann angesehen, wenn die Zubereitung eine Viskosität von 200 bis 3000 mPas bei einer Temperatur von 25 °C gemessen mit Rheomat 123 von proRheo mit Spindel 1 (Messbereich 300-10000 mPas) aufweist.

Es ist erfindungsgemäß vorteilhaft, wenn die Zubereitung 1 bis 2 Gewichts-% Glycin, bezogen auf das Gesamtgewicht der Zubereitung enthält. Dabei ist es erfindungsgemäß bevorzugt, wenn die Zubereitung von 1 bis 1,5 Gewichts-% Glycin, bezogen auf das Gesamtgewicht der Zubereitung enthält.

Erfindungsgemäß vorteilhafte Ausführungsformen der vorliegenden Erfindung sind ferner dadurch gekennzeichnet, dass die Zubereitung Ethylendiamintetraessigsäure (EDTA) und/oder dessen Natriumsalz in einer Gesamtmenge von 0,3 bis 0,6 Gewichts-%, bezogen auf das Gesamtgewicht der Zubereitung, enthält.

Erfindungsgemäß bevorzugte Ausführungsformen der vorliegenden Erfindung sind ferner dadurch gekennzeichnet, dass die Zubereitung Ethylendiamintetraessigsäure (EDTA) und/oder dessen Natriumsalz in einer Gesamtmenge von 0,3 bis 0,4 Gewichts-%, bezogen auf das Gesamtgewicht der Zubereitung, enthält.

Es ist vorteilhaft im Sinne der vorliegenden Erfindung, wenn die Zubereitung 1,2-Alkandiole in einer Gesamtmenge von 2 bis 6 Gewichts-%, bezogen auf das Gesamtgewicht der Zubereitung, enthält. Die erfindungsgemäß bevorzugte Einsatzkonzentration an 1,2-Alkandiolen ist dabei eine Gesamtmenge von 2,8 bis 4,3 Gewichts-%, bezogen auf das Gesamtgewicht der Zubereitung.

Es ist erfindungsgemäß vorteilhaft, wenn die erfindungsgemäße Zubereitung 1,2-Alkandiole gewählt aus der Gruppe der Verbindungen Propylenglycol und Butylenglycol enthält.

Weiterhin ist es erfindungsgemäß vorteilhat, wenn die erfindungsgemäße Zubereitung 1,2-Alkandiole gewählt aus der Gruppe der Verbindungen 2-Methylpropan-1,3-diol, 1,2-Pentandiol, 1,2-Hexandiol, 1,2-Octandiol und/oder 1,2-Decandiol enthält.

Erfindungsgemäß vorteilhaft sind darüber hinaus Kombinationen aus zwei oder mehreren 1,2-Alkandiolen, u.a. die Kombinationen aus Propylenglycol bzw. Butylenglycol mit 1,2-Octandiol.

Enthält die erfindungsgemäße Zubereitung eines der erfindungsgemäßen 1,2-Alkandiole, so sind die folgenden Konzentrationsbereiche erfindungsgemäß vorteilhaft:
Propylenglycol: von 2 bis 3 Gewichts-%, bezogen auf das Gesamtgewicht der Zubereitung.
Butylenglycol: von 2 bis 4 Gewichts-%, bezogen auf das Gesamtgewicht der Zubereitung.
2-Methylpropan-1,3-diol: von 2 bis 2,5 Gewichts-%, bezogen auf das Gesamtgewicht der Zubereitung.
1,2-Pentandiol: von 0,1 bis.0,3 Gewichts-%, bezogen auf das Gesamtgewicht der Zubereitung.
1,2-Hexandiol: von 0,1 bis 0,3 Gewichts-%, bezogen auf das Gesamtgewicht der Zubereitung.
1,2-Octandiol: von 0,1 bis 0,2 Gewichts-%, bezogen auf das Gesamtgewicht der Zubereitung.
1,2-Decandiol von 0,1 bis 0,2 Gewichts-%, bezogen auf das Gesamtgewicht der Zubereitung.

Erfindungsgemäß vorteilhafte Ausführungsformen der vorliegenden Erfindung sind dadurch gekennzeichnet, dass die Zubereitung 4-(tert.-Butyl)-4'-methoxydibenzoylmethan und/oder Hexyl 2-[4-(diethylamino)-2-hydroxybenzoyl]benzoate (INCI: Diethylamino hydroxybenzoyl hexyl benzoate) enthält.

Enthält die erfindungsgemäße Zubereitung 4-(tert.-Butyl)-4'-methoxydibenzoylmethan, so ist es erfindungsgemäß vorteilhaft, wenn 4-(tert.-Butyl)-4'-methoxydibenzoylmethan in einer Menge von 2 bis 4,75 Gewichts-%, bezogen auf das Gesamtgewicht der Zubereitung in dieser enthalten ist.

Enthält die erfindungsgemäße Zubereitung Hexyl 2-[4-(diethylamino)-2-hydroxybenzoyl]-benzoate (INCI: Diethylamino hydroxybenzoyl hexyl benzoate), so ist es erfindungsgemäß vorteilhaft, wenn diese Verbindung in einer Menge von 4 bis 9 Gewichts-%, bezogen auf das Gesamtgewicht der Zubereitung in dieser enthalten ist.

Es ist erfindungsgemäß vorteilhaft, wenn die erfindungsgemäße Zubereitung ein oder mehrere UV-Filter mit dem Grundgerüst des Triazins enthält. Dabei ist es erfindungsgemäß bevorzugt, wenn die Zubereitung ein oder mehrere Triazinderivate gewählt aus der Gruppe der Verbindungen 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) und 4,4'-[[6-[[4-[[(1,1-dimethylethyl)amino]carbonyl]phenyl]amino]-1,3,5-triazine-2,4-diyl]diimino]bis(2-ethylhexyl)benzoat (INCI: Diethylhexyl Butamido Triazone) enthält.

Enthält die erfindungsgemäße Zubereitung 2,4,6-Tris-[anilino-(p-carbo-2'-ethyl-1'-hexyloxy)]-1,3,5-triazin (INCI: Ethylhexyl Triazone), so ist es erfindungsgemäß vorteilhaft, dieses Triazinderivat in einer Konzentration von 2 bis 4 Gewichts-%, bezogen auf das Gesamtgewicht der Zubereitung einzusetzen.

Enthält die erfindungsgemäße Zubereitung 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine), so ist es erfindungsgemäß vorteilhaft, dieses Triazinderivat in einer Konzentration von 1 bis 4 Gewichts-%, bezogen auf das Gesamtgewicht der Zubereitung einzusetzen.

Enthält die erfindungsgemäße Zubereitung 4,4'-[[6-[[4-[[(1,1-dimethylethyl)amino]-carbonyl]phenyl]amino]-1,3,5-triazine-2,4-diyl]diimino]bis(2-ethylhexyl)benzoat (INCI: Diethylhexyl Butamido Triazone), so ist es erfindungsgemäß vorteilhaft, dieses Triazinderivat in einer Konzentration von 1 bis 2 Gewichts-%, bezogen auf das Gesamtgewicht der Zubereitung einzusetzen.

Darüber hinaus sind erfindungsgemäß vorteilhafte Ausführungsformen der vorliegenden Erdfindung dadurch gekennzeichnet, dass die Zubereitung 2-Phenylbenzimidazol-5-sulfonsäure und/oder deren Salze enthält.

In einem solchen Falle ist es erfindungsgemäß von Vorteil, wenn die Zubereitung 2-Phenylbenzimidazol-5-sulfonsäure und/oder deren Salze in einer Gesamtkonzentration von 0,5 bis 2,0 Gewichts-%, bezogen auf das Gesamtgewicht der Zubereitung, enthält.

Erfindungsgemäß vorteilhafte Ausführungsformen werden auch dadurch erhalten, dass die Zubereitung Ethylhexylsalicylat und/oder Homomenthylsalicylat enthält.

Enthält die erfindungsgemäße Zubereitung Homomenthylsalicylat, so ist es erfindungsgemäß vorteilhaft, wenn Homomenthylsalicylat in einer Menge von 4 bis 9 Gewichts-%, bezogen auf das Gesamtgewicht der Zubereitung, in dieser enthalten ist.

Enthält die erfindungsgemäße Zubereitung Ethylhexylsalicylat, so ist es erfindungsgemäß vorteilhaft, wenn Ethylhexylsalicylat in einer Menge von 2 bis 4,75 Gewichts-%, bezogen auf das Gesamtgewicht der Zubereitung, in dieser enthalten ist.

Erfindungsgemäß vorteilhafte Ausführungsformen der vorliegenden Erfindung sind auch dadurch gekennzeichnet, dass die Zubereitung einen oder mehrere weitere UV-Filter enthält, die gewählt werden aus der Gruppe der Verbindungen Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)-sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Terephthalidendicamphersulfonsäure; 2-Ethylhexyl-2-cyano-3,3-diphenylacrylat; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone; 2,4,6-Tribiphenyl-4-yl-1,3,5-triazin; Merocyanine; Titandioxid; Zinkoxid.

Es ist vorteilhaft im Sinne der vorliegenden Erfindung, wenn die erfindungsgemäße Zubereitung Ethylhexylglycerin und/oder Phenoxyethanol enthält.

Enthält die erfindungsgemäße Rezeptur Ethylhexylglycerin, so ist es erfindungsgemäß vorteilhaft, wenn diese Verbindung in einer Konzentration von 0,1 bis 0,3 Gewichts-%, bezogen auf das Gesamtgewicht der Zusammensetzung enthalten ist.

Enthält die erfindungsgemäße Rezeptur Phenoxyethanol, so ist es erfindungsgemäß vorteilhaft, wenn diese Verbindung in einer Konzentration von 0,2 bis 0,5 Gewichts-%, bezogen auf das Gesamtgewicht der Zusammensetzung enthalten ist.

Erfindungsgemäß vorteilhafte Ausführungsformen der vorliegenden Erfindung sind auch dadurch gekennzeichnet, dass die Zubereitung Xanthangummi und/oder Acrylat/C10-30 Alkylacrylat-Crosspoymer und/oder Microcrystalline Cellulose in Kombination mit Cellulose Gum enthält

Enthält die erfindungsgemäße Rezeptur Xanthangummi, so ist es erfindungsgemäß vorteilhaft, wenn diese Verbindung in einer Konzentration von 0,05 bis 0,4 Gewichts-%, bezogen auf das Gesamtgewicht der Zusammensetzung enthalten ist.

Enthält die erfindungsgemäße Rezeptur Acrylat/C10-30 Alkylacrylat-Crosspoymer, so ist es erfindungsgemäß vorteilhaft, wenn diese Verbindung in einer Konzentration von 0,1 bis 0,4 Gewichts-%, bezogen auf das Gesamtgewicht der Zusammensetzung enthalten ist.

Enthält die erfindungsgemäße Rezeptur Microcrystalline Cellulose in Kombination mit Cellulose Gum, so ist es erfindungsgemäß vorteilhaft, wenn diese Verbindung in einer Konzentration von 0,1 bis 1,5 Gewichts-%, bezogen auf das Gesamtgewicht der Zusammensetzung enthalten ist.

Erfindungsgemäß vorteilhaft sind auch Ausführungsformen, die dadurch gekennzeichnet sind, dass die Zubereitung Vinylpyrrolidon-Hexadecen-Copolymer enthält.

Enthält die erfindungsgemäße Rezeptur Vinylpyrrolidon-Hexadecen-Copolymer, so ist es erfindungsgemäß vorteilhaft, wenn diese Verbindung in einer Konzentration von 0,5 bis 1,5 Gewichts-%, bezogen auf das Gesamtgewicht der Zusammensetzung enthalten ist.

Erfindungsgemäß vorteilhaft liegt die erfindungsgemäße Zubereitung in Form einer O/W-Emulsion vor.

Darüber hinaus ist es erfindungsgemäß von Vorteil, wenn die Zubereitung mit einem der Emulgatorsysteme
a) Ceteareth-20 oder
b) einem Emulgatorsystem enthaltend Sodium Stearoyl Glutamate, Sucrose Polystearate in Kombination mit Hydrogenated Polyisobutene, Glyceryl Stearate Citrate, Cetearylalkohol und/oder Glyceryl Stearate
   emulgiert ist.

Enthält die erfindungsgemäße Rezeptur Ceteareth-20 als Emulgator, so ist es erfindungsgemäß vorteilhaft, wenn diese Verbindung in einer Konzentration von 0,5 bis 1,5 Gewichts-%, bezogen auf das Gesamtgewicht der Zusammensetzung enthalten ist.

Enthält die erfindungsgemäße Rezeptur das Emulgatorsystem aus Sodium Stearoyl Glutamate, Sucrose Polystearate in Kombination mit Hydrogenated Polyisobutene, Glyceryl Stearate Citrate, Cetearylalkohol und/oder Glyceryl Stearate, so ist es erfindungsgemäß vorteilhaft, wenn die Zubereitung
Sodium Stearoyl Glutamate in einer Konzentration von 0,1 bis 0,6 Gewichts-%, bezogen auf das Gesamtgewicht der Zusammensetzung enthält,
Sucrose Polystearate in Kombination mit Hydrogenated Polyisobutene in einer Konzentration von 0,5 bis 1,5 Gewichts-%, bezogen auf das Gesamtgewicht der Zusammensetzung enthält, Cetearylalkohol in einer Konzentration von 0,5 bis 1,5 Gewichts-%, bezogen auf das Gesamtgewicht der Zusammensetzung enthält,
Glyceryl Stearate Citrate in einer Konzentration von 1,0 bis 3,0 Gewichts-%, bezogen auf das Gesamtgewicht der Zusammensetzung enthält sowie
Glycerylstearat in einer Konzentration von 0,5 bis 1,5 Gewichts-%, bezogen auf das Gesamtgewicht der Zusammensetzung enthält.

Die wässrige Phase einer erfindungsgemäßen Emulsion kann die für solche Zubereitungen üblichen Inhaltsstoffe enthalten, insbesondere Wasser, Ethanol und in diesen Inhaltsstoffen lösliche Komponenten.

Die Ölphase einer erfindungsgemäßen Emulsion kann ebenfalls die für solche Zubereitungen üblichen Öl-Wachs- und Lipidkomponenten enthalten.

Erfindungsgemäß bevorzugt ist dabei ein Gehalt an C18-36-Fettsäuretriglyceriden, C12-15 Alkylbenzoaten und hydrierten Cocosglyceriden, sowie Dicaprylylcarbonat, und Butylenglycol Dicaprylat/Dicaprat

Erfindungsgemäß vorteilhafte Ausführungsformen der vorliegenden Erfindung sind ferner dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, ß-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

Es ist ferner vorteilhaft im Sinne der vorliegenden Erfindung, wenn die erfindungsgemäße Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Nicht zuletzt ist es erfindungsgemäß bevorzugt, wenn die erfindungsgemäße Zubereitung frei ist von Parabenen, Methylisothiazolinon, Chlormethylisothiazolinon, DMDM-Hydantoin, 3-(4-Methylbenzyliden)-campher und 2-Hydroxy-4-methoxybenzophenon (INCI: Oxybenzon).

Erfindungsgemäß ist auch ein Pumpspender enthaltend eine erfindungsgemäße Zubereitung, sowie eine Aerosoldose enthaltend eine erfindungsgemäße Zubereitung.

Die erfindungsgemäßen Zubereitungen werden erfindungsgemäß besonders bevorzugt als Sonnenschutzsprays eingesetzt.

Erfindungsgemäß ist nicht zuletzt die Verwendung sowohl von Glycin als auch von Ethylendiamintetraessigsäure (EDTA) und/oder dessen Natriumsalz, zur Regulierung der Tröpfchenverteilung von versprühbaren kosmetische Zubereitungen insbesondere O/W-Emulsionen.

### Vergleichsversuch

Mit dem folgenden Versuch konnte der erfindungsgemäße Effekt beispielhaft belegt werden: Der Versuch wurde mit der Pumpe PZ2/190 DL D1 Helios 5027 von dem Hersteller APTAR durchgeführt. Im Abstand von 10cm wurde das Sprühbild hergestellt.

Tröpfchenverteilung von Formel 1: Sprühbild Ø: 1-2cm ein Strahl (Jet) - keine Verteilung.

Tröpfchenverteilung von Formel 2: Sprühbild Ø: 5-6cm mittelbreites Sprühbild leicht strahlig.

| Inhaltsstoffe | Formel 1 | Formel 2 |
|---|---|---|
| Ethylhexylglycerin | 0,25 | 0,25 |
| Glycine | 2 | 2 |
| Butylene Glycol Dicaprylate/Dicaprate | 3 | 3 |
| C18-36 Acid Triglyceride | 1 | 1 |
| Ceteareth-20 | 1,3 | 1,3 |
| VP/Hexadecene Copolymer | 1 | 1 |
| Glycerin | 5 | 5 |
| Butylene Glycol | 4 | 4 |
| Sodium Hydroxide 45%ig | 0,65 | 0,65 |
| Acrylates/C10-30 Alkyl Acrylate Crosspolymer | 0,3 | 0,3 |
| Aqua | ad 100 | ad 100 |
| Alcohol Denat. | 6 | 6 |
| Trisodium EDTA | 0,2 | 0,3 |
| Homosalate | 9 | 9 |
| Octocrylene | 8 | 8 |
| Ethylhexyl Salicylate | 4,5 | 4,5 |
| Bis-Ethylhexyloxyphenol Methoxyphenyl Triazine | 3,5 | 3,5 |
| Butyl Methoxydibenzoylmethane | 4,5 | 4,5 |
| Phenylbenzimidazole Sulfonic Acid | 1 | 1 |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| inci | A | B | C | D | E |
|---|---|---|---|---|---|
| Ceteareth-20 | | 1,5 | | | 0,5 |
| Sodium Stearoyl Glutamate | 0,4 | | | 0,35 | |
| Sucrose Polystearate in Kombination mit Hydroqenated Polyisobutene | 1 | | | | 0,5 |
| Glyceryl Stearate | | | | 1,2 | |
| Glyceryl Stearate Citrate | | | 2 | | |
| Cetearylalkohol | | | 1,2 | | |
| Octocrylene | | 9 | | | |
| Homomenthylsalicylat | | 9 | 4 | | |
| Ethylhexyl Salicylate | | 4,75 | 2 | | 4,5 |
| Diethylhexyl Butamido Triazone | | | 1 | | 2 |
| Bis-Ethylhexyloxyphenol Methoxyphenyl Triazine | 3,5 | 4 | | | 1 |
| Butyl Methoxydibenzoylmethane | 2 | 4,75 | 3 | | |
| Phenylbenzimidazole Sulfonic Acid | | 2 | | 0,5 | 1 |
| Ethylhexyl Triazone | | | 2 | | 4 |
| Diethylamino Hydroxybenzoyl Hexyl Benzoate | | | | 4 | 9 |
| Polysilicone-15 | | | | 1 | |
| C18-36 Acid Triglyceride | 0,5 | 1 | | | |
| Butylene Glycol Dicaprylate/Dicaprate | | 4 | | | |
| C12-15 Alkylbenzoaten | 9 | | | | |
| Aqua | ad 100 | ad 100 | ad 100 | ad 100 | ad 100 |
| Sodium Hydroxide 45%ig | | 0,69 | 0,1 | 0,57 | |
| VP/Hexadecene Copolymer | 0,5 | 1 | 0,8 | 0,75 | 1,5 |
| Xanthangummi | 0,2 | | | 0,05 | 0,4 |
| Acrylates/C10-30 Alkyl Acrylate Crosspolymer | | 0,3 | 0,1 | 0,4 | |
| Microcrystalline Cellulose in Kombination mit Cellulose Gum | 0,75 | | | | |
| Glycerin | 7,5 | 5 | 8 | 9 | 10 |
| Trisodium EDTA | 0,5 | 0,3 | 0,4 | 0,35 | 0,6 |
| Glycine | 1,5 | 1,2 | 1,3 | 1 | 2 |
| Alcohol Denat. + Aqua | 4 | 6 | 5 | 5 | 6 |
| Butylene Glycol | | 2 | | | 4 |
| Propylene Glycol | 2 | | 3 | | |
| Ethylhexylglycerin | | 0,3 | | 0,1 | 0,25 |
| Phenoxylethanol | 0,4 | | 0,2 | | |
| 2-Methylpropan-1,3-diol | | 2,5 | | 2 | |
| 1,2-Decandiol | 0,2 | | | | |
| 1,2-Octandiol | 0,2 | | | | |
| 1,2-Pentandiol | 0,1 | | | 0,3 | |
| 1,2-Hexandiole | | | 0,1 | 0,4 | |

## Patentansprüche

1. Kosmetische Zubereitung enthaltend
a) ein oder mehrere UV-Lichtschutzfilter,
b) Glycin,
c) Ethylendiamintetraessigsäure (EDTA) und/oder dessen Natriumsalz,
d) ein oder mehrerer 1,2-Alkandiole.

2. Kosmetische Zubereitung nach Anspruch 1, **dadurch gekennzeichnet, dass** die Zubereitung sprühbar ist.

3. Kosmetische Zubereitung nach einem der vorhergehenden Ansprüche, **dadurch gekennzeichnet, dass** die Zubereitung 1 bis 2 Gewichts-% Glycin, bezogen auf das Gesamtgewicht der Zubereitung enthält.

4. Kosmetische Zubereitung nach einem der vorhergehenden Ansprüche, **dadurch gekennzeichnet, dass** die Zubereitung Ethylendiamintetraessigsäure (EDTA) und/oder dessen Natriumsalz in einer Gesamtmenge von 0,3 bis 0,6 Gewichts-%, bezogen auf das Gesamtgewicht der Zubereitung, enthält.

5. Kosmetische Zubereitung nach einem der vorhergehenden Ansprüche, **dadurch gekennzeichnet, dass** die Zubereitung 1,2-Alkandiole in einer Gesamtmenge von 2 bis 6 Gewichts-%, bezogen auf das Gesamtgewicht der Zubereitung, enthält.

6. Kosmetische Zubereitung nach einem der vorhergehenden Ansprüche, **dadurch gekennzeichnet, dass** die Zubereitung eine Viskosität von 200 bis 3000 mPas bei einer Temperatur von 25 °C gemessen mit Rheomat 123 von proRheo mit Spindel 1 (Messbereich 300-10000 mPas) aufweist.

7. Kosmetische Zubereitung nach einem der vorhergehenden Ansprüche, **dadurch gekennzeichnet, dass** die Zubereitung 4-(tert.-Butyl)-4'-methoxydibenzoylmethan und/oder Hexyl 2-[4-(diethylamino)-2-hydroxybenzoyl]benzoate (INCI: Diethylamino hydroxybenzoyl hexyl benzoate) enthält.

8. Kosmetische Zubereitung nach einem der vorhergehenden Ansprüche, **dadurch gekennzeichnet, dass** die Zubereitung ein oder mehrere Triazinderivate gewählt aus der Gruppe der Verbindungen 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) und 4,4'-[[6-[[4-[[(1,1-dimethylethyl)amino]carbonyl]phenyl]amino]-1,3,5-triazine-2,4-diyl]diimino]bis(2-ethylhexyl)benzoat (INCI: Diethylhexyl Butamido Triazone) enthält.

9. Kosmetische Zubereitung nach einem der vorhergehenden Ansprüche, **dadurch gekennzeichnet, dass** die Zubereitung 2-Phenylbenzimidazol-5-sulfonsäure und/oder deren Salze enthält.

10. Kosmetische Zubereitung nach einem der vorhergehenden Ansprüche, **dadurch gekennzeichnet, dass** die Zubereitung Ethylhexylsalicylat und/oder Homomenthylsalicylat enthält.

11. Kosmetische Zubereitung nach einem der vorhergehenden Ansprüche, **dadurch gekennzeichnet, dass** die Zubereitung Ethylhexylglycerin und/oder Phenoxyethanol enthält.

12. Kosmetische Zubereitung nach einem der vorhergehenden Ansprüche, **dadurch gekennzeichnet, dass** die Zubereitung 1,2-Alkandiole gewählt aus der Gruppe der Verbindungen Propylenglycol und Butylenglycol enthält.

13. Kosmetische Zubereitung nach einem der vorhergehenden Ansprüche, **dadurch gekennzeichnet, dass** die Zubereitung 1,2-Alkandiole gewählt aus der Gruppe der Verbindungen 2-Methylpropan-1,3-diol, 1,2-Pentandiol, 1,2-Hexandiol, 1,2-Octandiol und/oder 1,2-Decandiol enthält.

14. Kosmetische Zubereitung nach einem der vorhergehenden Ansprüche, **dadurch gekennzeichnet, dass** die Zubereitung Xanthangummi, Acrylat/C10-30 Alkylacrylat-Crosspoymer und/oder Microcrystalline Cellulose in Kombination mit Cellulose Gum enthält.

15. Kosmetische Zubereitung nach einem der vorhergehenden Ansprüche, **dadurch gekennzeichnet, dass** die Zubereitung Vinylpyrrolidon-Hexadecen-Copolymer enthält.

16. Kosmetische Zubereitung nach einem der vorhergehenden Ansprüche, **dadurch gekennzeichnet, dass** die Zubereitung mit einem der Emulgatorsysteme
a) Ceteareth-20 oder
b) einem Emulgatorsystem enthaltend Sodium Stearoyl Glutamate, Sucrose Polystearate in Kombination mit Hydrogenated Polyisobutene, Glyceryl Stearate Citrate, Cetearylalkohol und/oder Glyceryl Stearate emulgiert ist.

17. Kosmetische Zubereitung nach einem der vorhergehenden Ansprüche, **dadurch gekennzeichnet, dass** die Zubereitung frei ist von Parabenen, Methylisothiazolinon, Chlormethylisothiazolinon, DMDM-Hydantoin, 3-(4-Methylbenzyliden)-campher und 2-Hydroxy-4-methoxybenzophenon (INCI: Oxybenzon).
